# EUROPEAN PATENT APPLICATION

(11) **EP 4 374 769 A1**
(43) Date of publication of application: **29.05.2024**
(21) Application number: 22846253.7
(22) Date of filing: 21.07.2022
(51) Int. Cl.: A61B 1/24, A61B 1/00, A61B 5/00, A61C 9/00

(54) **METHOD AND APPARATUS FOR ADJUSTING SCAN DEPTH OF THREE-DIMENSIONAL SCANNER**

(30) Priority: 21.07.2021 KR 20210095453; 19.05.2022 KR 20220061439
(71) Applicant: Medit Corp., Seoul 07207 (KR)
(72) Inventor: LEE, Dong Hoon, Seoul 07207 (KR)
(74) Representative: MFG Patentanwälte Meyer-Wildhagen Meggle-Freund Gerhard PartG mbB
(86) International application number: PCT/KR2022/010687
(87) International publication number: WO 2023/003383

(57) **Abstract**

As one aspect of the present disclosure, a method and apparatus for adjusting a scan depth of a three-dimensional scanner may be suggested.

A method according to one aspect of the present disclosure is a method for processing a scan image of a three-dimensional scanner, which is performed by at least one processor of an electronic device comprising the at least one processor and at least one memory configured to store instructions to be executed by the at least one processor, wherein the method may comprises: acquiring, through the three-dimensional scanner, scan data of a measurement object within a preconfigured scan depth of the three-dimensional scanner; determining whether the scan data is abnormal based on a scan image acquired from the three-dimensional scanner; and adjusting the scan depth of the three-dimensional scanner in a case of determining that there is abnormality in the scan data.

## Description

### TECHNICAL FIELD

The present disclosure relates to a method and apparatus for adjusting a scan depth of a three-dimensional scanner. Specifically, the present disclosure relates to a method and apparatus for analyzing data scanned through a three-dimensional scanner and automatically adjusting a scan depth of the scanner accordingly.

### BACKGROUND

A three-dimensional scanner may scan an object existing within a certain distance from a part (e.g., a probe tip of the scanner) that scans a subject. In the present disclosure, such a scannable distance is referred to as a "scan depth." A user (e.g., a dentist) of the three-dimensional scanner may adjust the depth by changing a configuration of the scanner as needed.

When the scan depth is configured to be deep, that is, a value of the scan depth is configured to be large, the scannable region becomes greater and more data may be acquired through scanning, but the possibility of generating noise may increase. For example, it is possible to scan a deeper region of the subject, and thus even data of a region (e.g., an inside of an oral cavity) that the user of the three-dimensional scanner does not intend to scan may be acquired. Furthermore, since the scanning is possible even when the scanner is positioned away from the subject, the possibility that an object (e.g., a finger of the user, a treatment tool, or the like) other than the subject intervenes between the scanner and the subject may increase.

Conversely, when the scan depth is configured to be shallow, that is, a value of the scan depth is configured to be small, the possibility of generating noise may be reduced, but it may be difficult to generate an entire three-dimensional image due to reduced data acquired, and it may cause inconvenience of having to bring the scanner closer to the subject. Therefore, it is necessary to determine an appropriate depth depending on a situation.

Conventionally, in order to change the scan depth of the scanner, the user must manually adjust the scan depth through a user interface displayed on a display using a mouse, a touch screen, or the like. However, when the user directly touches the mouse or the display to adjust the scan depth, for example, during a dental procedure, there is a possibility of contaminating the user's hand that may enter the patient's oral cavity.

In addition, since the user needs to directly manipulate the display or the mouse to change the scan depth, the user may be subject to the inconvenience of having to increase movement especially when the distance between a treatment position and an input device is far, and accordingly, it may cause problems that the measurement time of an intraoral scanner is increased and patient's fatigue caused by the increased measurement time is increased as well.

### SUMMARY

An aspect of the present disclosure is to solve the aforementioned problems of the conventional art and may prevent contamination that may occur due to a user directly manipulating an input device and allow convenience and fast scanning by adjusting a scan depth of an intraoral scanner according to a preconfigured condition. Further, the present disclosure may allow the scan depth to be automatically adjusted to smoothly adjust a distance between the scanner and the subject and automatically filter out parts (e.g., an oral soft tissue or the like) that are not subject to scanning.

As one aspect of the present disclosure, a method and apparatus for adjusting a scan depth of a three-dimensional scanner may be suggested.

A method according to one aspect of the present disclosure is a method for processing a scan image of a three-dimensional scanner, which is performed by at least one processor of an electronic device comprising the at least one processor and at least one memory configured to store instructions to be executed by the at least one processor, wherein the method may comprises: acquiring, through the three-dimensional scanner, scan data of a measurement object within a preconfigured scan depth of the three-dimensional scanner; determining whether the scan data is abnormal based on a scan image acquired from the three-dimensional scanner; and adjusting the scan depth of the three-dimensional scanner in a case of determining that there is abnormality in the scan data.

As one aspect of the present disclosure, an electronic device for adjusting a scan depth of a three-dimensional scanner may be suggested. An electronic device according to one aspect of the present disclosure comprises a communication circuit communicatively connected to a three-dimensional scanner; a display; and at least one processor, wherein the at least one processor may be configured to: acquire, through the three-dimensional scanner, scan data of a measurement object within a preconfigured scan depth of the three-dimensional scanner; determine whether the scan data is abnormal based on a scan image acquired from the three-dimensional scanner; and adjust the scan depth of the three-dimensional scanner in a case of determining that there is abnormality in the scan data.

As one aspect of the present disclosure, a non-transitory computer-readable recording medium for storing instructions for adjusting a scan depth of a three-dimensional scanner may be suggested. A non-transitory computer-readable recording medium, according to one aspect of the present disclosure, for storing instructions configured to, when executed by at least one processor, cause the at least one processor to perform an operation, wherein the instructions may cause the at least one processor to: acquire, through a three-dimensional scanner, scan data of a measurement object within a preconfigured scan depth of the three-dimensional scanner; determine whether the scan data is abnormal based on a scan image acquired from the three-dimensional scanner; and adjust the scan depth of the three-dimensional scanner in a case of determining that there is abnormality in the scan data.

According to at least one embodiment of a method and apparatus for adjusting a scan depth of the present disclosure, contamination which may occur due to a user directly manipulating an input device may be prevented, and convenient and fast scanning may be possible by automatically adjusting the scan depth of a three-dimensional scanner based on scan data. Further, according to at least one embodiment of the method and apparatus for adjusting a scan depth of the present disclosure, when scanning of a non-target part is being performed, the user is notified of same and unnecessary scanning and data processing may be prevented by reducing the scan depth.

### BRIEF DESCRIPTION OF DRAWINGS

FIG. 1 is a view illustrating an operation of acquiring an image of a patient's oral cavity by using a three-dimensional scanner according to various embodiments of the present disclosure.
FIG. 2A is a block view illustrating an electronic device and a three-dimensional scanner according to various embodiments of the present disclosure. FIG. 2B is a perspective view illustrating a three-dimensional scanner according to various embodiments of the present disclosure.
FIG. 3 is a view illustrating a method for generating a three-dimensional image of an oral cavity according to various embodiments of the present disclosure.
FIG. 4 is a view illustrating a scan depth of a three-dimensional scanner according to various embodiments of the present disclosure.
FIG. 5 is a view illustrating a screen of a data acquisition program displayed on a display of an electronic device according to various embodiments of the disclosure.
FIGS. 6A to 6C are views illustrating a case in which a value of a scan depth is increased or reduced.
FIG. 7 is a flowchart illustrating a method for automatically adjusting a scan depth value of a three-dimensional scanner according to various embodiments of the present disclosure.
FIG. 8 is a view illustrating that a portion used for generating three-dimensional data and a portion not used for generating three-dimensional data from among images acquired by a three-dimensional scanner are separately displayed according to various embodiments of the present disclosure.
FIG. 9 is a flowchart illustrating a method for automatically adjusting a scan depth in a predetermined scan mode according to various embodiments of the present disclosure.
FIG. 10 is a view illustrating an image in which a target object and a non-target object are separated and masked in an acquired two-dimensional image according to various embodiments of the present disclosure.

### DETAILED DESCRIPTION

Embodiments of the present disclosure are illustrated for describing the technical idea of the present disclosure. The scope of the claims according to the present disclosure is not limited to the embodiments described below or to the detailed descriptions of these embodiments.

All technical or scientific terms used in the present disclosure have meanings that are generally understood by a person having ordinary knowledge in the art to which the present disclosure pertains, unless otherwise specified. The terms used in the present disclosure are selected for the purpose of clearer explanation of the present disclosure, and are not intended to limit the scope of claims according to the present disclosure.

The expressions "include," "provided with," "have" and the like used in the present disclosure should be understood as open-ended terms connoting the possibility of inclusion of other embodiments, unless otherwise mentioned in a phrase or sentence including the expressions.

A singular expression used in the present disclosure can include meanings of plurality, unless otherwise mentioned, and the same is applied to a singular expression stated in the claims. The terms "first," "second," etc. used the present disclosure are used to distinguish a plurality of elements from one another, and are not intended to limit the order or importance of the relevant elements.

The term "unit" used in the present disclosure means a software element or hardware element, such as a field-programmable gate array (FPGA) or an application specific integrated circuit (ASIC). However, a "unit" is not limited to software and hardware. A "unit" may be configured to be stored in an addressable storage medium or may be configured to run on one or more processors. Therefore, for example, a "unit" may include elements, such as software elements, object-oriented software elements, class elements, and task elements, as well as processors, functions, attributes, procedures, subroutines, segments of program codes, drivers, firmware, micro-codes, circuits, data, databases, data structures, tables, arrays, and variables. Functions provided in elements and "unit" may be combined into a smaller number of elements and "units" or further subdivided into additional elements and "units."

The expression "based on" used in the present disclosure is used to describe one or more factors that influences a decision, an action of determination, or an operation described in a phrase or sentence including the relevant expression, and this expression does not exclude an additional factor influencing the decision, the action of determination, or the operation.

In the present disclosure, when a certain element is described as being "coupled to" or "connected to" another element, it should be understood that the certain element may be connected or coupled directly to the other element or that the certain element may be connected or coupled to the other element via a new intervening element.

In the present disclosure, artificial intelligence (AI) may refer to a technology that imitates human learning ability, reasoning ability, and perception ability and implements the same with a computer, and may include concepts of machine learning and symbolic logic. Machine learning (ML) may be an algorithmic technique that automatically classifies or learns the characteristics of input data. Artificial intelligence technology may use machine learning algorithms to analyze input data, learn the results of the analysis, and make determinations or predictions based on the results of the learning. Further, technologies that mimic the cognitive and decision-making functions of the human brain by using machine learning algorithms may also be considered to fall within the realm of artificial intelligence. For example, technical fields of language understanding, visual comprehension, reasoning and prediction, knowledge representation, and action control may be included.

In the present disclosure, machine learning may refer to a process of training a neural network model by using experience in processing data. Machine learning may imply that computer software improves its ability to process data on its own. A neural network model is constructed by modeling correlations between data, wherein the correlations may be represented by multiple parameters. The neural network model extracts features from given data and analyzes the features to derive correlations between data, and repeating this process to optimize the parameters of the neural network model may be referred to as machine learning. For example, a neural network model can learn the mapping (correlation) between inputs and outputs with respect to data provided as input-output pairs. Alternatively, even when only input data is provided, a neural network model can learn the relationships between the provided data by deriving regularities in the data.

In the present disclosure, an artificial intelligence learning model, a machine learning model, or a neural network model may be designed to implement a human brain structure on a computer and may include multiple network nodes that mimic neurons in a human neural network and have weights. Multiple network nodes may have connections to one another by mimicking the synaptic activity of neurons sending and receiving signals through synapses. In an artificial intelligence learning model, multiple network nodes can be located on layers having different depths to send and receive data based on convolutional connections. The artificial intelligence learning model may be, for example, an artificial neural network, a convolutional neural network, etc.

Hereinafter, embodiments of the present disclosure will be described with reference to the accompanying drawings. In the accompanying drawings, identical or corresponding elements are indicated by identical reference numerals. In the following description of embodiments, repeated descriptions of the identical or corresponding elements will be omitted. However, even when a description of an element is omitted, such an element is not intended to be excluded in an embodiment.

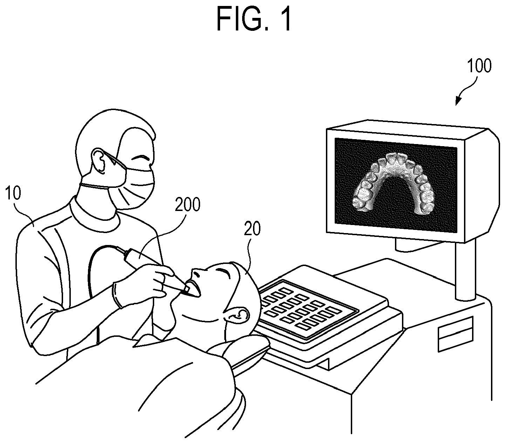

FIG. 1 illustrates acquiring an image of a patient's oral cavity by using an three-dimensional scanner 200 according to various embodiments of the present disclosure. According to various embodiments, the three-dimensional scanner 200 may be a dental medical device for acquiring an image of an oral cavity of a subject 20. For example, the three-dimensional scanner 200 may include an intraoral scanner. As illustrated in FIG. 1, a user 10 (e.g., a dentist or a dental hygienist) may use the three-dimensional scanner 200 to acquire an image of the oral cavity of a subject 20 (e.g., a patient) from the subject 20. In another example, the user 10 may acquire an image of the oral cavity of the subject 20 from a diagnostic model (e.g., a plaster model or an impression model) that is made in the shape of the oral cavity of the subject 20. Hereinafter, for ease of explanation, it will be described that an image of the oral cavity of the subject 20 is acquired by scanning the oral cavity of the subject 20. However, the present disclosure is not limited thereto, and it is also possible to acquire an image of another part of the subject 20 (e.g., an ear of the subject 20). The three-dimensional scanner 200 may be shaped to be inserted into or removed from the oral cavity, and may be a handheld scanner for which a scanning distance and a scanning angle can be freely adjusted by the user 10.

The three-dimensional scanner 200 according to various embodiments may be inserted into the oral cavity of the subject 20 and scan the interior of the oral cavity in a non-contact manner, thereby acquiring an image of the oral cavity. The image of the oral cavity may include at least one tooth, gingiva, and artificial structures insertable into the oral cavity (e.g., orthodontic devices including brackets and wires, implants, dentures, and orthodontic aids inserted into the oral cavity). The three-dimensional scanner 200 may use a light source (or a projector) to emit light to the oral cavity of the subject 20 (e.g., at least one tooth or gingiva of the subject 20), and may receive light reflected from the oral cavity of the subject 20 through a camera (or at least one image sensor). According to another embodiment, the three-dimensional scanner 200 may scan a diagnostic model of the oral cavity to acquire an image of the diagnostic model of the oral cavity. When the diagnostic model of the oral cavity is a diagnostic model that mimics the shape of the oral cavity of subject 20, an image of the diagnostic model of the oral cavity may be an image of the oral cavity of the subject. For ease of explanation, the following description assumes, but is not limited to, acquiring an image of the oral cavity by scanning the inside of the oral cavity of the subject 20.

The three-dimensional scanner 200 according to various embodiments may acquire a surface image of the oral cavity of the subject 20 as a two-dimensional image based on information received through a camera. The surface image of the oral cavity of the subject 20 may include at least one among at least one tooth, gingiva, artificial structure, cheek, tongue, or lip of the subject 20. The surface image of the oral cavity of subject 20 may be a two-dimensional image.

The two-dimensional image of the oral cavity acquired by the three-dimensional scanner 200 according to various embodiments may be transmitted to an electronic device 100 which is connected via a wired or wireless communication network. The electronic device 100 may be a computer device or a portable communication device. The electronic device 100 may generate a three-dimensional image (or, three-dimensional oral cavity image or a three-dimensional oral model) of the oral cavity, which is a three-dimensional representation of the oral cavity, based on the two-dimensional images of the oral cavity received from the three-dimensional scanner 200. The electronic device 100 may generate the three-dimensional image of the oral cavity by modeling the inner structure of the oral cavity in three dimensions, based on the received two-dimensional images of the oral cavity.

The three-dimensional scanner 200 according to another embodiment may scan the oral cavity of the subject 20 to acquire two-dimensional images of the oral cavity, generate a three-dimensional image of the oral cavity based on the acquired two-dimensional images of the oral cavity, and transmit the generated three-dimensional image of the oral cavity to the electronic device 100.

The electronic device 100 according to various embodiments may be communicatively connected to a cloud server (not shown). In this case, the electronic device 100 may transmit two-dimensional images of the oral cavity of the subject 20 or a three-dimensional image of the oral cavity to the cloud server, and the cloud server may store the two-dimensional images of the oral cavity of the subject 20 or the three-dimensional image of the oral cavity, which has been received from the electronic device 100.

According to another embodiment, in addition to a handheld scanner that is inserted into the oral cavity of subject 20, a table scanner (not shown) which is fixed and used in a specific location may be used as the three-dimensional scanner. The table scanner can generate a three-dimensional image of a diagnostic model of the oral cavity by scanning the diagnostic model of the oral cavity. In the above case, since a light source (or projector) and a camera of the table scanner are fixed, the user may scan the oral diagnosis model while moving an arm for fixing the oral diagnosis model. In comparison to a handheld scanner, although the table scanner may have a relatively lower possibility of causing noise due to intervention by other objects between the camera and the diagnosis model during the scanning operation, the embodiments of the present disclosure are applicable not only to handheld scanners, but also to table scanners and other three-dimensional scanners.

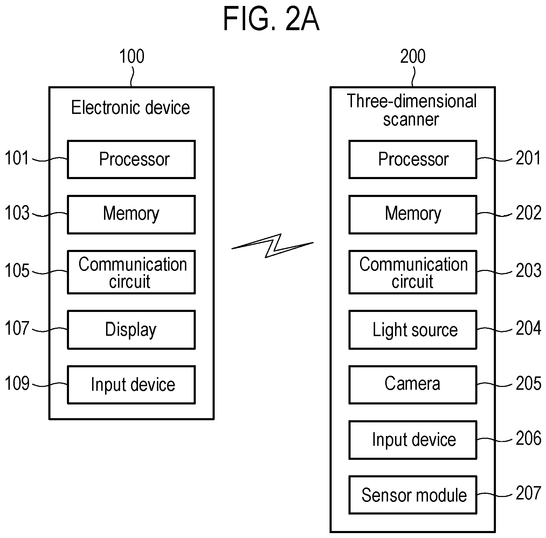

FIG. 2A is a block diagram of an electronic device 100 and an three-dimensional scanner 200 according to various embodiments of the present disclosure. The electronic device 100 and the three-dimensional scanner 200 may be communicatively connected to each other via a wired or wireless communication network, and may transmit and receive various types of data to and from each other.

The three-dimensional scanner 200 according to various embodiments may include a processor 201, a memory 202, a communication circuit 203, a light source 204, a camera 205, an input device 206, and/or a sensor module 207. At least one of the elements included in the three-dimensional scanner 200 may be omitted, or other elements may be added to the three-dimensional scanner 200. Additionally or alternatively, some elements may be integrated, or implemented as a single or multiple entities. At least some elements in the three-dimensional scanner 200 may be connected to each other via a bus, a general purpose input/output (GPIO), a serial peripheral interface (SPI), or a mobile industry processor interface (MIPI) so as to transmit and/or receive data and/or signals.

The processor 201 of the three-dimensional scanner 200 according to various embodiments is an element capable of performing operations or data processing related to control and/or communication of the elements of the three-dimensional scanner 200, and may be operatively coupled to the elements of the three-dimensional scanner 200. The processor 201 may load commands or data received from other elements of the three-dimensional scanner 200 into the memory 202, may process the commands or data stored in the memory 202, and may store resulting data. According to various embodiments, the memory 202 of the three-dimensional scanner 200 may store instructions for the above-described operations of the processor 201.

According to various embodiments, the communication circuit 203 of the three-dimensional scanner 200 may establish a wired or wireless communication channel with an external device (e.g., the electronic device 100), and may transmit and receive various types of data to and from the external device. According to an embodiment, for wired communication with the external device, the communication circuit 203 may include at least one port to be connected to the external device with a wired cable. In the above case, the communication circuit 203 may communicate with the external device that is connected to a wire via the at least one port. According to an embodiment, the communication circuit 203 may be configured to include a cellular communication module so as to be connected to a cellular network (e.g., 3G, LTE, 5G, Wibro, or Wimax). In various embodiments, the communication circuit 203 may communicate with an external device by using a millimeter wave frequency band, for example, a communication band of 30 GHz to 300 GHz. For example, the communication circuit 203 may communicate with an external device through a wireless network using a frequency band of 60 GHz. Furthermore, according to various embodiments, the communication circuit 203 may include a short-range communication module to transmit and receive data to and from external devices by using short-range communication (e.g., Wi-Fi, Bluetooth, Bluetooth Low Energy (BLE), UWB), but the present disclosure is not limited thereto. According to an embodiment, the communication circuit 203 may include a contactless communication module for contactless communication. The contactless communication may include at least one contactless proximity communication technology, for example, near-field communication (NFC) communication, radio frequency identification (RFID) communication, or magnetic secure transmission (MST) communication.

According to various embodiments, the light source 204 of the three-dimensional scanner 200 may emit light toward the oral cavity of the subject 20. For example, the light emitted from the light source 204 may be structured light having a predetermined pattern (e.g., a stripe pattern in which differently colored straight lines are continuously shown). The pattern of the structured light may be generated, for example, using a pattern mask or a digital micro-mirror device (DMD), but the present disclosure is not limited thereto. The camera 205 of the three-dimensional scanner 200 according to various embodiments may acquire an image of the oral cavity of the subject 20 by receiving light reflected by the oral cavity of the subject 20. The camera 205 may include, for example, a left camera corresponding to the left field of view and a right camera corresponding to the right field of view in order to construct a three-dimensional image by using optical triangulation. The camera 205 may include at least one image sensor, such as a CCD sensor or a CMOS sensor.

The input device 206 of the three-dimensional scanner 200 according to various embodiments may receive a user input for controlling the three-dimensional scanner 200. The input device 206 may include buttons for receiving push manipulation from the user 10, a touch panel for detecting touch from the user 10, and a speech recognition device including a microphone. For example, the user 10 may use the input device 206 to control the start or stop of scanning.

The sensor module 207 of the three-dimensional scanner 200 according to various embodiments may detect an operational state of the three-dimensional scanner 200 or an external environmental state (e.g., a user's motion) and generate an electrical signal corresponding to the detected state. The sensor module 207 may include, for example, at least one of a gyro sensor, an accelerometer, a gesture sensor, a proximity sensor, or an infrared sensor. The user 10 may use the sensor module 207 to control the start or stop of scanning. For example, when the user 10 is moving while holding the three-dimensional scanner 200 in hand, the three-dimensional scanner 200 may be controlled to start a scanning operation of the processor 201 when an angular velocity measured by the sensor module 207 exceeds a predetermined threshold.

According to an embodiment, the three-dimensional scanner 200 may start scanning by receiving a user input for starting scanning through the input device 206 of the three-dimensional scanner 200 or an input device 206 of the electronic device 100, or in response to processing in a processor 201 of the three-dimensional scanner 200 or the processor 201 of the electronic device 100. When the user 10 scans the inside of the oral cavity of the subject 20 by using the three-dimensional scanner 200, the three-dimensional scanner 200 may generate a two-dimensional image of the oral cavity of the subject 20, and may transmit the two-dimensional image of the oral cavity of the subject 20 to the electronic device 100 in real time. The electronic device 100 may display the received two-dimensional image of the oral cavity of the subject 20 on a display. Further, the electronic device 100 may generate (construct) a three-dimensional image of the oral cavity of the subject 20, based on the two-dimensional images of the oral cavity of the subject 20, and may display the three-dimensional image of the oral cavity on the display. The electronic device 100 may display the three-dimensional image, which is being generated, on the display in real time.

The electronic device 100 according to various embodiments may include at least one processor 101, at least one memory 103, a communication circuit 105, a display 107, and/or an input device 109. At least one of the elements included in the electronic device 100 may be omitted, or other elements may be added to the electronic device 100. Additionally or alternatively, some elements may be integrated, or implemented as a single or multiple entities. At least some elements in the electronic device 100 may be connected to each other via a bus, a general purpose input/output (GPIO), a serial peripheral interface (SPI), or a mobile industry processor interface (MIPI) so as to exchange data and/or signals.

According to various embodiments, the at least one processor 101 of the electronic device 100 may be an element capable of performing operations or data processing related to control and/or communication of the elements of the electronic device 100 (e.g., memory 103). The at least one processor 101 may be operatively coupled to the elements of the electronic device 100, for example. The at least one processor 101 may load commands or data received from other elements of the electronic device 100 into the at least one memory 103, may process the commands or data stored in the at least one memory 103, and store the resulting data.

According to various embodiments, the at least one memory 103 of the electronic device 100 may store instructions for operations of the at least one processor 101. The at least one memory 103 may store correlation models constructed based on a machine learning algorithm. The at least one memory 103 may store data (e.g., a two-dimensional image of the oral cavity acquired through oral scanning) received from the three-dimensional scanner 200.

According to various embodiments, the communication circuit 105 of the electronic device 100 may establish a wired or wireless communication channel with an external device (e.g., the three-dimensional scanner 200 or the cloud server), and may transmit or receive various types of data to or from the external device. According to an embodiment, for wired communication with the external device, the communication circuit 105 may include at least one port so as to be connected to the external device through a wired cable. In the above case, the communication circuit 105 may communicate with the external device that is connected to a wire via the at least one port. According to an embodiment, the communication circuit 105 may be configured to include a cellular communication module so as to be connected to a cellular network (e.g., 3G, LTE, 5G, Wibro, or Wimax). According to various embodiments, the communication circuit 105 may include a short-range communication module to transmit and receive data to and from external devices by using short-range communication (e.g., Wi-Fi, Bluetooth, Bluetooth Low Energy (BLE), or UWB), but the present disclosure is not limited thereto. According to an embodiment, the communication circuit 105 may include a contactless communication module for contactless communication. The contactless communication may include at least one contactless proximity communication technology, for example, near-field communication (NFC) communication, radio frequency identification (RFID) communication, or magnetic secure transmission (MST) communication.

The display 107 of the electronic device 100 according to various embodiments may display various screens based on control of the processor 101. The processor 101 may display, on the display 107, a two-dimensional image of the oral cavity of subject 20 received from the three-dimensional scanner 200 and/or a three-dimensional image of the oral cavity in which the inner structure of the oral cavity is modeled. For example, the two-dimensional image and/or the three-dimensional image of the oral cavity may be displayed through a particular application. In the above case, the user 10 may edit, save, and delete the two-dimensional image and/or the three-dimensional image of the oral cavity.

The input device 109 of the electronic device 100 according to various embodiments may receive commands or data, which are to be used by an element (e.g., the at least one processor 101) of the electronic device 100, from an external source (e.g., a user) of the electronic device 100. The input device 109 may include, for example, a microphone, a mouse, or a keyboard. According to an embodiment, the input device 109 may be implemented in the form of a touch sensor panel that may be coupled to the display 107 to recognize contact or proximity of various external objects.

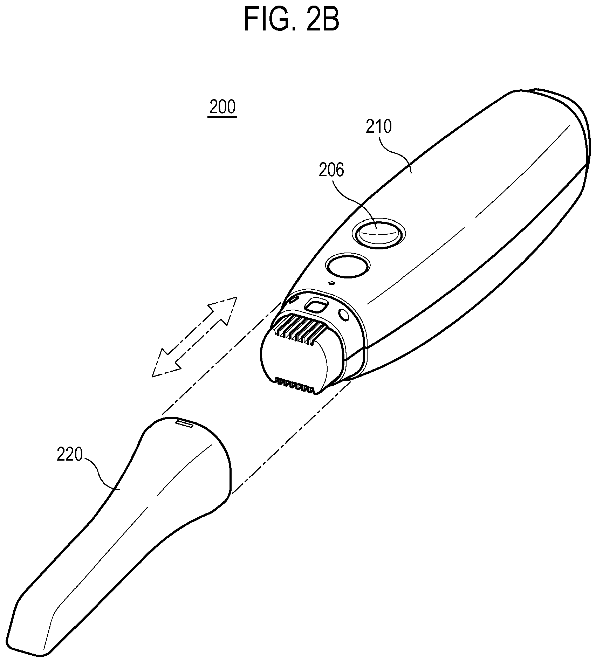

FIG. 2B is a perspective view of an three-dimensional scanner 200 according to various embodiments. The three-dimensional scanner 200 according to various embodiments may include a body 210 and a probe tip 220. The body 210 of the three-dimensional scanner 200 may be formed in a shape that is easy for the user 10 to grip and use by hand. The probe tip 220 may be shaped for easy insertion into and removed from the oral cavity of the subject 20. Further, the body 210 may be coupled to and detachable from the probe tip 220. Inside the body 210, the elements of the three-dimensional scanner 200 illustrated in FIG. 2A may be disposed. An opening may be formed at one end of the body 210 so that light output from the light source 204 can be emitted onto the subject 20 through the opening. Light emitted through the opening may be reflected by the subject 20 and introduced again through the opening. The reflected light introduced through the opening may be captured by the camera to generate an image of the subject 20. The user 10 may start scanning by using the input device 206 (e.g., a button) of the three-dimensional scanner 200. For example, when the user 10 touches or presses the input device 206, light from the light source 204 may be emitted onto the subject 20.

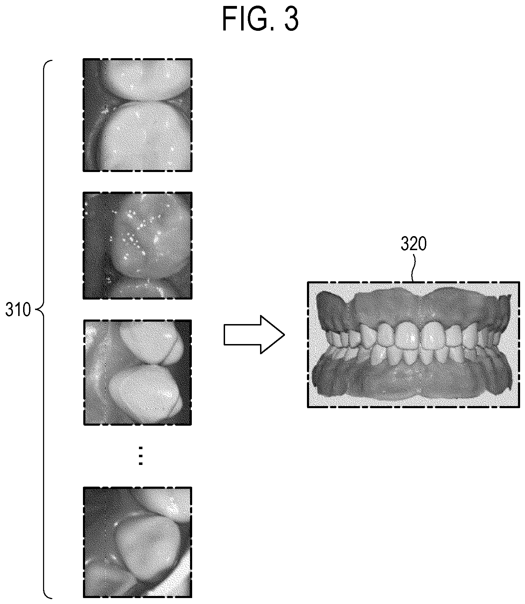

FIG. 3 illustrates a method for generating a three-dimensional image 320 of an oral cavity according to various embodiments. The user 10 may move the three-dimensional scanner 200 to scan the inside of the oral cavity of the subject 20, in which case the three-dimensional scanner 200 may acquire multiple two-dimensional images 310 of the oral cavity of the subject 20. For example, the three-dimensional scanner 200 may acquire a two-dimensional image of a region including a front tooth of the subject 20, a two-dimensional image of a region including a molar of the subject 20, and the like. The three-dimensional scanner 200 may transmit the multiple acquired two-dimensional images 310 to the electronic device 100. According to another embodiment, the user 10 may scan a diagnostic model of the oral cavity while moving the three-dimensional scanner 200, or acquire multiple two-dimensional images of the diagnostic model of the oral cavity. Hereinafter, for convenience of explanation, a case in which an image of the oral cavity of the subject 20 is acquired by scanning the inside of the oral cavity of the subject 20 is assumed, but the embodiments are not limited thereto.

According to various embodiments, the electronic device 100 may convert each of the multiple two-dimensional images 310 of the oral cavity of the subject 20 into a set of multiple points having three-dimensional coordinate values. For example, the electronic device 100 may convert each of the multiple two-dimensional images 310 into a point cloud, which is a set of data points having three-dimensional coordinate values. For example, a set of point clouds, which are three-dimensional coordinate values based on the multiple two-dimensional images 310, may be stored as raw data for the oral cavity of subject 20. By aligning the point clouds, each of which is a set of data points having three-dimensional coordinate values, the electronic device 100 may complete a full dental model.

According to various embodiments, the electronic device 100 may reconfigure (reconstruct) a three-dimensional image of the oral cavity. For example, the electronic device 100 may reconfigure the three-dimensional image 320 of the oral cavity of the subject 20 by merging a set of point clouds stored as raw data by using a Poisson algorithm to reconfigure multiple points and transforming the points into a closed three-dimensional surface.

As described above, the three-dimensional scanner 200 may acquire an image of the oral cavity by being inserted into the oral cavity of the subject 20 and scanning the oral cavity in a non-contact manner. Here, a scannable distance of the three-dimensional scanner 200, that is, a distance at which data may be acquired by scanning, is referred to as a "scan depth."

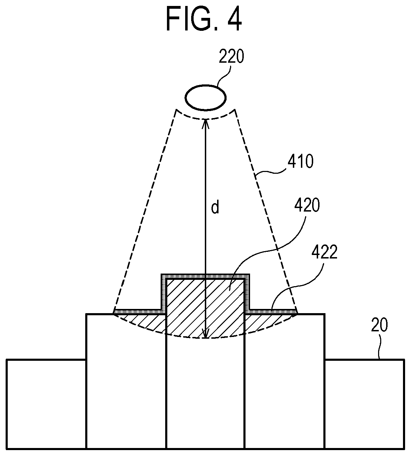

FIG. 4 illustrates a scan depth of a three-dimensional scanner 200 according to various embodiments of the present disclosure. FIG. 4 illustrates a scan region 410 when the three-dimensional scanner 200 having a scan depth d scans the subject 20. In an embodiment, a scannable distance d from a camera portion of the three-dimensional scanner 200, for example, the probe tip 220, corresponds to the scan depth. In an embodiment of FIG. 4, a portion 420 of the subject 20 belongs to the scan region 410, and a surface 422 of the subject belonging to the scan region 410 may be acquired as a two-dimensional image.

In an embodiment, the scan depth refers to a distance configured by software so that the camera 205 of the three-dimensional scanner 200 acquires data within a maximum image capturing distance. Here, from data actually acquired by the camera, only data belonging to the scan depth may be used for subsequent data processing, and adjusting the scan depth may include adjusting a distance to data used for subsequent data processing by using software. In another embodiment, the scan depth refers to a distance at which the camera 205 of the three-dimensional scanner 200 may capture an image. In this case, all data actually acquired by the camera may be used for subsequent data processing, and adjusting the scan depth may include adjusting hardware of the camera 205 of the three-dimensional scanner 200 to adjust a distance at which the camera 205 may capture an image. In the present disclosure, unless otherwise specified, the term "scan depth" means a distance at which the three-dimensional scanner may acquire data, and may include both of the above cases. For example, when the scan depth value is 18.5 mm, data up to a distance of 18.5 mm from the scan portion (probe tip) of the three-dimensional scanner is acquired for subsequent processing.

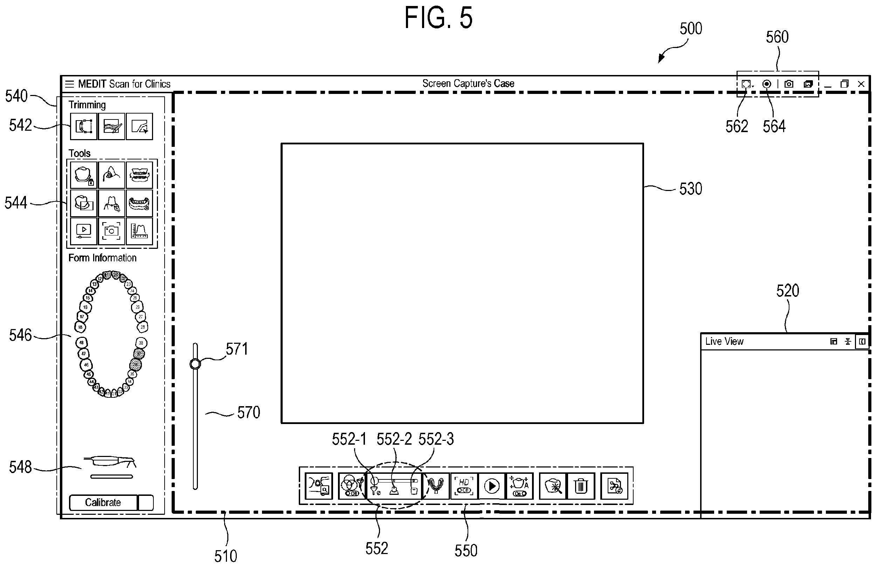

FIG. 5 is a view illustrating a screen 500 of a data acquisition program displayed on a display 107 of an electronic device 100 according to various embodiments of the disclosure. The electronic device 100 may execute the data acquisition program by using the processor 100. The data acquisition program corresponds to an application program and may be stored in the memory 103. When the data acquisition program is executed by the processor 101, the electronic device 100 may display the two-dimensional images 310 received from the intraoral scanner 200 and a three-dimensional image generated on the basis thereof on the display 107.

When the data acquisition program is executed, the electronic device 100 may display images acquired by the intraoral scanner 100 and provide a user interface for processing the images. In an embodiment, the screen 500 of the data acquisition program includes user interfaces including a data display area 510, a live view area 520, a model view area 530, a function box area 540, a function option area 550, and an icon display area 560. The above user interfaces are merely exemplary, and any additional user interfaces may be included in the screen 500 of the data acquisition program as needed.

The data display area 510 is an area for displaying an image received from the intraoral scanner and a three-dimensional image generated based thereon. In an embodiment, the data display area 510 includes the live view area 520 and the model view area 530. In an embodiment, the data display area 510 includes the function option area 550.

The live view area 520 displays an image received from the intraoral scanner 100. In an embodiment, the electronic device 100 may display a two-dimensional image of the oral cavity currently being scanned by the intraoral scanner 200 in the live view area 520 in real time. The live view area 520 may be moved and resized by the user, and may be separated from the screen 500 of the data acquisition program. In an embodiment, the user may configure the live view area 520 not to be displayed.

The electronic device 100 may display a three-dimensional image model generated from two-dimensional images acquired from the intraoral scanner 100 on the model view area 530.

The function box area 540 includes a user interface for providing a function for modifying/editing or analyzing the displayed three-dimensional image model and a user interface for displaying a device state. In an embodiment, the function box area 540 includes a trimming function interface 542 for selecting and deleting an unnecessary data portion acquired during the scanning process, a function tool interface 544 for editing or saving a generated three-dimensional image, a treatment information interface 546 for displaying an image of treatment information for each tooth, and a device state display interface 548 for displaying an apparatus state of the intraoral scanner 200.

The electronic device 100 may display, in response to a user input for selecting one function interface from the function box area 540, detailed options thereof in the function option area 550. In an embodiment, when the user selects a playback control interface of the function tool interface 544 of the function box area 550, the electronic device 100 may display an option for playback of the recorded screen in the function option area 550, for example, a play/stop button, an interface for controlling playback position/speed, and the like. The function options area 550 may include a scan filtering function option 552. The scan filtering function includes a function of generating a scanned image by automatically removing unnecessary parts (e.g., soft tissues in the oral cavity) during scanning. In an embodiment, when a no-filtering mode 552-1 of the scan filtering function option 552 is selected, unnecessary parts such as soft tissues are not removed from the data acquired by scanning, and all parts are generated as images. When a tooth and gingiva mode 552-2 of the scan filtering function option 552 is selected, only data corresponding to the tooth and the gingival portion adjacent to the tooth is retained while data corresponding to the soft tissue portion is removed. When a tooth mode 552-3 of the scan filtering function option 552 is selected, only data corresponding to the tooth is retained and the remaining data is removed. Whether the acquired data corresponds to a tooth, gingiva, or a soft tissue may be automatically determined using an AI learning model trained using intraoral images as learning data. When a function that does not require detailed options is selected in the function box area 540, the function option area 550 may not be displayed.

The icon display area 560 is an area for providing a function for screen recording and capture, and may include a recording area configuration icon 562 and a recording start/end icon 564. The recording area configuration icon 562 provides an interface for configuring a screen area to be recorded. The recording start/end icon 564 provides an interface for starting or ending recording of the screen 400 of the data acquisition program.

A scan depth control bar 570 provides an interface for adjusting the scan depth of the three-dimensional scanner 200. By manipulating a scan depth control button 571 of the scan depth control bar 570, the user may increase or decrease the scan depth of the three-dimensional scanner. Upper and lower ends of the scan depth control bar 570 may indicate maximum and minimum values of a current depth range, and a position of the scan depth control button 571 may indicate a current scan depth value. A numerical value (e.g., 0 mm) or a percentage (e.g., 0%) corresponding to the minimum value of the scan depth may be displayed on the lower portion of the scan depth control bar 570, and a numerical value (e.g., 24 mm) or a percentage (e.g., 100%) corresponding to the maximum value of the scan depth may be displayed on the upper portion thereof. In an embodiment, the user may increase the scan depth value by moving the scan depth adjustment button 571 upward through the input device 206, and may decrease the scan depth value by moving the scan depth adjustment button 571 downward.

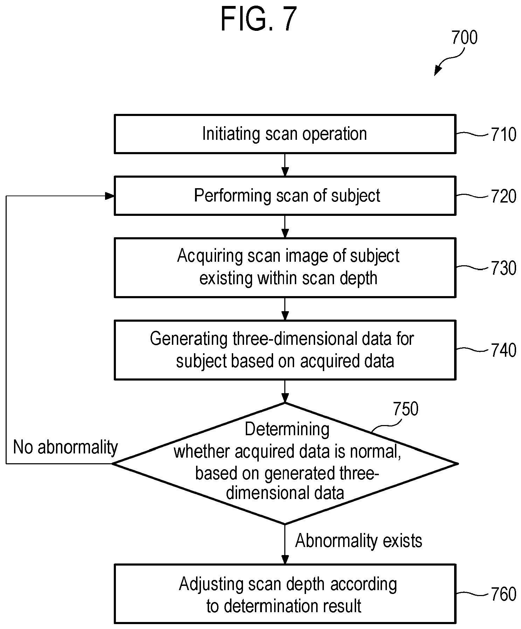

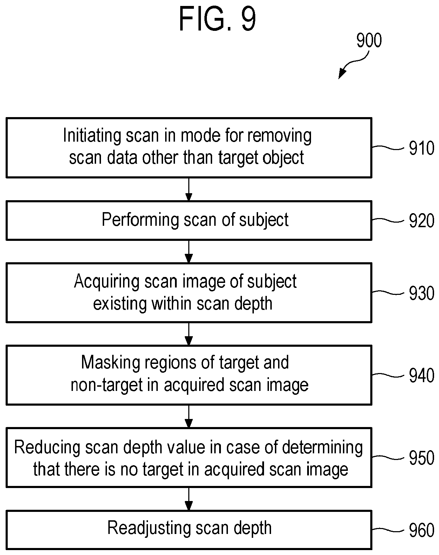

In an embodiment, as will be described below with reference to FIGS. 7 and 9, when the scan depth value of the three-dimensional scanner 200 is automatically adjusted, the scan depth adjustment bar 570 may be adjusted to reflect the automatically adjusted scan depth value. For example, the scan depth control button 571 of the scan depth control bar 570 may move to a position corresponding to the automatically adjusted scan depth value in real time.

In an embodiment, although not shown in FIG. 5, the data acquisition program 500 may further include a user interface capable of turning on/off a function of automatically adjusting the scan depth value of the three-dimensional scanner 200 as described below. In an additional embodiment, when the user manually adjusts the scan depth, a function of automatically adjusting the scan depth value of the three-dimensional scanner 200 as described below may be turned off, that is, released. For example, when the user adjusts the scan depth value by using the scan depth adjustment button 571 or the like, a user interface capable of turning on/off a function of automatically adjusting the above scan depth value may be turned off.

The above-described user interface of the data acquisition program 500 is merely an example and additional user interfaces and/or other types of user interfaces may be included in the data acquisition program.

The user of the three-dimensional scanner 200 may adjust a basic value of the scan depth or the scan depth range by changing a configuration of the scanner as needed. In an embodiment, a basic scan depth value of the three-dimensional scanner may be 18.5 mm, and a range of the scan depth value may be configured from a minimum of 0 mm to a maximum of 12 to 24 mm, but is not limited thereto. In an embodiment, the user may configure an adjustable scan depth value range within the scan depth value range allowed by the three-dimensional scanner 200. For example, in case that the scan depth value range allowed by the three-dimensional scanner 200 falls within 0 to 24 mm, the user may configure, through the user interface of the data acquisition program 500, the adjustable scan depth value range to be smaller than that, for example, to have a range of 0 to 12 mm, 5 to 12 mm, 8.5 to 15 mm, or other various ranges. Numerical values or percentages corresponding to the maximum and minimum values of the configured scan depth value range may be displayed on the scan depth control bar 570 as described above.

The scan depth value may be adjusted through the user interface of the data acquisition program 500. For example, the user may adjust the scan depth value of the three-dimensional scanner 200 by adjusting the scan depth adjustment button 571 through the input device 206, for example, a mouse or a touch panel. In an embodiment, the scan depth value may be increased by moving the scan depth adjustment button 571 upward, and the scan depth value may be decreased by moving the scan depth adjustment button 571 downward.

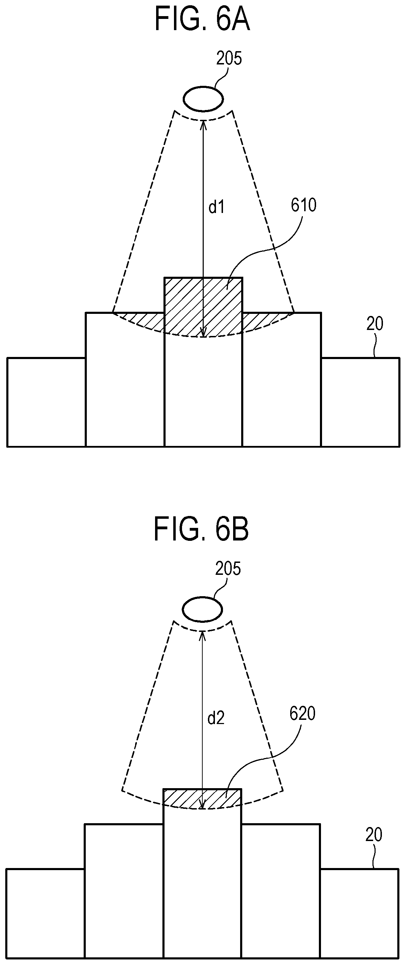

FIGS. 6A to 6C illustrate a case in which a value of a scan depth is increased or reduced. In an embodiment shown in FIG. 6A, the scan depth value is configured to d1. The scan depth value d1 may correspond to a basic scan depth value basically used when the three-dimensional scanner 200 operates. The basic scan depth value may be a predetermined setting value or may be a scan depth value last used in a previous scan of the three-dimensional scanner 200. In the embodiment shown in FIG. 6A, a surface of a portion 610 of the subject 20 belonging to the scan depth d1 may be acquired as a two-dimensional image.

FIG. 6B illustrates a case in which the scan depth value is reduced to d2. In an embodiment, the scan depth value may be reduced by moving the scan depth adjustment button 571 of the data acquisition program 500 downward. In the embodiment shown in FIG. 6B, a surface of a portion 620 of the subject 20 belonging to the scan depth d2 may be acquired as a two-dimensional image. Due to the reduced scan depth value d2, the portion 620 of the subject belonging to the scan depth d2 may be smaller than the portion 610 of the subject belonging to the scan depth d1 of FIG. 6A.

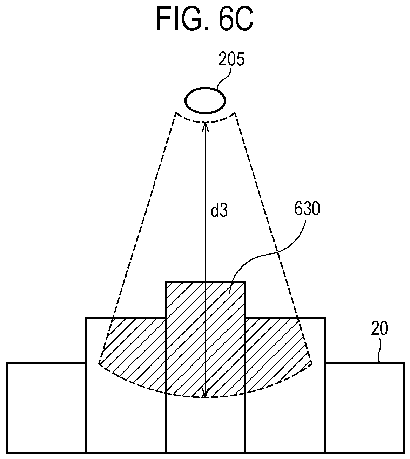

FIG. 6C illustrates a case in which the scan depth value is increased to d3. In an embodiment, the scan depth value may be increased by moving the scan depth adjustment button 571 of the data acquisition program 500 upward. In the embodiment shown in FIG. 6C, a surface of a portion 630 of the subject 20 belonging to the scan depth d3 may be acquired as a two-dimensional image. Due to the increased scan depth value d3, the portion 630 of the subject belonging to the scan depth d3 may be greater than the portion 610 of the subject belonging to the scan depth d1 of FIG. 6A.

Hereinafter, a method and apparatus for automatically adjusting a scan depth value of the three-dimensional scanner 200 according to various embodiments of the present disclosure will be described.

FIG. 7 is a flowchart illustrating a method 700 for automatically adjusting a scan depth value of a three-dimensional scanner 200 according to various embodiments of the present disclosure. At least a part of a filtering method according to the present disclosure may be a method implemented by a computer, for example, the electronic device 100. However, the filtering method according to the present disclosure is not limited thereto and may be implemented by other devices, for example, the three-dimensional scanner 200.

Although steps of the method according to the present disclosure are illustrated in a sequential order in the illustrated flowchart, the steps may be performed in any order that may be arbitrarily combined by the present disclosure, in addition to being performed sequentially. The description according to the present flowchart does not exclude changes or modifications to the method or algorithm, and does not imply that any step is necessary or desirable. In an embodiment, at least some of the steps may be performed in parallel, iteratively or heuristically. In an embodiment, at least some of the steps may be omitted or other steps may be added.

In operation 710, a scanning operation of the three-dimensional scanner 200 is initiated. The initiating of the scan operation may include initiating an operation of software associated with the three-dimensional scanner 200. When the scan operation is initiated, the three-dimensional scanner 200 may perform a scan with a basic scan depth value. In an embodiment, the basic scan depth value may be a predetermined setting value or may be a scan depth value last used in a previous scan of the three-dimensional scanner 200.

In step 720, a scan of the subject 20 by using the three-dimensional scanner 200 is performed. The subject may include an oral cavity or a diagnostic model (e.g., plaster, impression, or the like).

In step 730, a scan image of the subject is acquired from a measuring unit of the three-dimensional scanner, for example, the probe tip 220 of the three-dimensional scanner 200. In an embodiment, the acquired scan image is a two-dimensional image of the surface of the subject existing within the scan depth, and acquiring the scan image of the subject includes selecting and acquiring only image data existing within the scan depth from among image data of the subject acquired by the camera.

In an embodiment, the acquired two-dimensional scan image may be transmitted to the electronic device 100 and displayed on the live view area 520 of the data acquisition program 500. In this embodiment, among the images acquired by the three-dimensional scanner 200, a portion used for generating three-dimensional data and a portion not used for generating three-dimensional data may be separately displayed on the live view area 520.

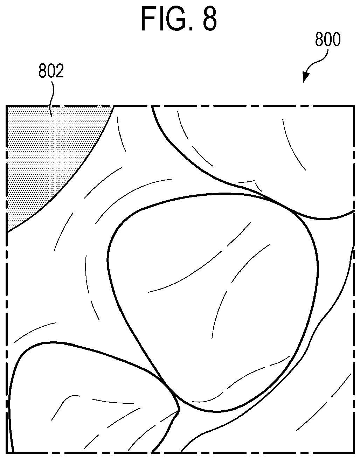

FIG. 8 illustrates that a portion used for generating three-dimensional data and a portion not used for generating three-dimensional data among images acquired by the three-dimensional scanner 200 are separately displayed on a live view area 800 according to various embodiments. The live view area 800 may correspond to the live view area 520 of the data acquisition program 500. In an embodiment shown in FIG. 8, a portion 802 not used for generating three-dimensional data is masked. In another embodiment, a portion used for generating three-dimensional data may be masked, or both the portion used and the portion not used for generating three-dimensional data may be masked in a separate manner.

The portion used for generating three-dimensional data and the portion not used for generating three-dimensional data may be classified according to various references. In an embodiment, the portion used for generating three-dimensional data corresponds to a region configured as a target. For example, the target object may be a region corresponding to a tooth or a region corresponding to a tooth and gingiva.

In an embodiment, the acquired scan image is a two-dimensional image acquired by the three-dimensional scanner regardless of the scan depth, and only scan images existing within the scan depth may be used as data acquired for generating a three-dimensional image. For example, in the embodiment shown in FIG. 8, all two-dimensional images acquired by the three-dimensional scanner may be displayed on the live view area 800, only data thereof within the scan depth configured by software may be used as data acquired for generating a three-dimensional image, and a region outside the scan depth may be masked and displayed as data not used for generating a three-dimensional image.

In step 740, three-dimensional data (three-dimensional image) of the subject is generated on the basis of the acquired data. In an embodiment, an align operation for connecting and aligning the generated three-dimensional data (e.g., voxels) is additionally performed. The generated three-dimensional data may be transmitted to the display 107 of the electronic device 100 and displayed on the model view area 530 of the data acquisition program 500.

In an embodiment, when the density of data (e.g., two-dimensional image data) acquired within the scan depth through operation 730 is lower than a preconfigured reference value (N%), for example, lower than N% of the live view area 520 on which the two-dimensional scan data is displayed, the acquired scan data is not applied to three-dimensional data generation. Accordingly, it is possible to prevent occurrence of an "align issue" in which three-dimensional data is not properly connected and aligned due to a scan region where less data was acquired.

In step 750, it is determined whether the acquired data is abnormal based on the generated three-dimensional data. In an embodiment, when information about a measurement object is not included in the three-dimensional data, that is, when the three-dimensional data is not generated, it is determined that there is an abnormality in the data as no object exists within a current scan depth. In an embodiment, if information about the measurement object is not included in the three-dimensional data for a predetermined time or for a predetermined number of scan operations, it is determined that there is an abnormality in the data as no object exists within the current scan depth.

Similarly, when the generated three-dimensional data lacks information about the measurement object, for example, when discontinuous data exists, holes occur, or the density of data is less than a predetermined reference value, it is determined that there is an abnormality in the data as an object has not sufficiently entered the current scan depth. In an embodiment, when information about the measurement object is insufficient in three-dimensional data generated over a predetermined time or a predetermined number of scan operations, it is determined that there is an abnormality in the data as an object has not sufficiently entered the current scan depth.

In an embodiment, when noise data is included in the generated three-dimensional data, it is determined that there is an abnormality in the data. The noise data refers to data generated by scanning an object other than the subject to be scanned, such as soft tissues such as a tongue, the inside of a cheek, etc., a finger, a treatment/diagnostic instrument, and other external foreign substances. Whether the generated data is noise data may be automatically determined by using an AI learning model trained using intraoral images as learning data.

In addition to the above cases in which it is determined that there is an abnormality in the data, additional cases in which inaccurate three-dimensional data may be generated may be further included, and in step 750, all of these cases may be determined to have an abnormality in the data. In case that it is determined that there is no abnormality in the data in step 750, the scan operation may continue to be performed by returning to step 720.

In step 760, in case that it is determined that there is an abnormality in the data, the scan depth of the three-dimensional scanner is automatically adjusted. The adjusting of the scan depth may include adjusting the scan depth value with software or adjusting the scan depth value with hardware. In an embodiment, in case that it is determined that no object exists within the current scan depth or that the object has not sufficiently entered the current scan depth, the scan depth value is increased to enable scanning of a longer distance. In an embodiment, the increasing of the scan depth value includes increasing the scan depth value by a preconfigured amount. In another embodiment, the increasing of the scan depth value includes changing the scan depth value to a preconfigured scan depth value, such as a maximum scan depth value.

As such, when the scan depth value is automatically increased, the subject sufficiently enters within the scan depth due to the increased scan depth, so that normal data acquisition is possible.

In an embodiment, when noise data is included in the generated three-dimensional data, it is determined that an object which is not a measurement target exists between the three-dimensional scanner and the subject, and the scan depth value is automatically reduced. In an embodiment, the reducing of the scan depth value includes reducing the scan depth value by a preconfigured amount. In another embodiment, the reducing of the scan depth value includes changing the scan depth value to a preconfigured scan depth value. Alternatively, a distance between the three-dimensional scanner and the object causing the noise may be determined on the basis of the noise data, and the scan depth value may be changed to a value smaller than the distance.

As such, when the scan depth value is automatically reduced, it is no longer possible to measure the subject at an existing position of the three-dimensional scanner due to the reduced scan depth. Accordingly, since the user is caused to move the three-dimensional scanner closer to the subj ect in order to narrow the distance between the subject and the three-dimensional scanner, it may be possible to prevent a situation in which noise occurs, for example, the user's hand or the patient's tongue or cheek, or the like intervening between the teeth and the scanner.

In an embodiment, in case that the scan depth value is automatically increased or reduced, the electronic device 100 or the three-dimensional scanner 200 may inform the user that the scan depth value is automatically changed through visual, auditory, and tactile notifications in various ways. For example, the electronic device 100 may display, through the data acquisition program 500, the change of the scan depth value to the user by using text or a picture/video on the screen, or inform the user by using a voice message, an alarm (e.g., a beep sound), or the like. Alternatively or additionally, vibration or an audible alarm may be provided to the user through the three-dimensional scanner 200. In an embodiment, a method of informing the user that the scan depth value is automatically changed may be different between a case of automatically increasing the scan depth value and a case of automatically increasing the scan depth value.

In an embodiment, in case of automatically increasing or reducing the scan depth value, as described above with reference to FIG. 5, the scan depth adjustment button 571 of the scan depth adjustment bar 570 of the data acquisition program 500 moves in real time to a position corresponding to the automatically adjusted scan depth value. In an embodiment, as described with reference to FIG. 5, when the user manually adjusts the scan depth, a function of automatically adjusting the scan depth value of the three-dimensional scanner 200 as described below may be turned off, that is, released.

Some or all of the above steps 720 to 760 may be performed at a predetermined time period or a predetermined scan (imaging) period. In an embodiment, steps 750 and 760 are not performed if the function to automatically adjust the scan depth value is turned off.

In the above embodiments, it has been described that the presence or absence of abnormality in data is determined based on the generated three-dimensional data, but the determination of whether the data is abnormal may be performed with respect to the two-dimensional image data acquired in step 730. For example, in step 750, it may be possible to determine whether the data is abnormal based on the two-dimensional image data acquired in step 730. According to this embodiment, when the acquired two-dimensional image data does not include information about the measurement object, or when the acquired two-dimensional image data lacks information about the measurement object, it is determined that the acquired data has an abnormality, and accordingly, the scan depth value is increased in operation 760. In addition, when noise data is included in the acquired two-dimensional image data, it is determined that there is an abnormality in the data, and accordingly, the scan depth value is reduced in operation 760.

In an embodiment, the determination of whether there is an abnormality in the data may be performed partly based on the two-dimensional image data and partly based on the three-dimensional data. For example, whether there is no or insufficient information about the measurement object in the acquired data may be determined based on the two-dimensional image data, and whether noise data is included may be determined based on the three-dimensional data, and vice versa.

According to various embodiments of the present disclosure, the scan depth value may be automatically adjusted according to a scan mode of the three-dimensional scanner and/or software (e.g., the data acquisition program 500) related to the three-dimensional scanner. In an embodiment, the scan mode may include three modes of the scan filtering function option 552 described with reference to FIG. 5, that is, a mode 552-1 in which unnecessary portions such as soft tissue are not removed from the data acquired by scanning, a mode 552-2 in which data corresponding to the soft tissue portion is removed while leaving only data corresponding to the tooth and the gingival portion adjacent to the tooth, and a mode 552-3 in which only data corresponding to the tooth is left and data of remaining portions is removed.

Among the above scan modes, in a mode for removing scan data other than the target object, such as the mode 552-2 for leaving teeth and gingiva and the mode 552-3 for leaving only teeth, when scan data of the target object is not acquired, it may be necessary to automatically adjust the scan depth.

FIG. 9 is a flowchart illustrating a method 900 for automatically adjusting a scan depth in a predetermined scan mode according to various embodiments of the present disclosure.

In step 910, a scan operation of the three-dimensional scanner 200 is initiated, and a mode for removing scan data other than the target object is selected.

In step 920, a scan of the subject 20 by using the three-dimensional scanner 200 is performed.

In step 930, a scan image of the subject existing within the scan depth is acquired from a measuring unit of the three-dimensional scanner, for example, the probe tip 220 of the three-dimensional scanner 200. In an embodiment, the acquired scan image is a two-dimensional image of a surface of a subject existing within the scan depth. In an embodiment, the acquired two-dimensional scan image may be transmitted to the electronic device 100 and displayed on the live view area 520 of the data acquisition program 500.

In step 940, in the acquired two-dimensional image, a target object region and a non-target object region are separated and masked. For example, when the mode 552-2 for leaving the teeth and gingiva is selected, the teeth and gingiva portions and other parts are separated and masked. Alternatively, when the mode 552-3 for leaving only teeth is selected, the teeth portion and other portions are separated and masked. In an embodiment, only the target object may be masked or only a portion other than the target object may be masked.

Whether the acquired data is the target object (teeth or gingiva) or other soft tissues may be automatically determined using an AI learning model trained using intraoral images as learning data.

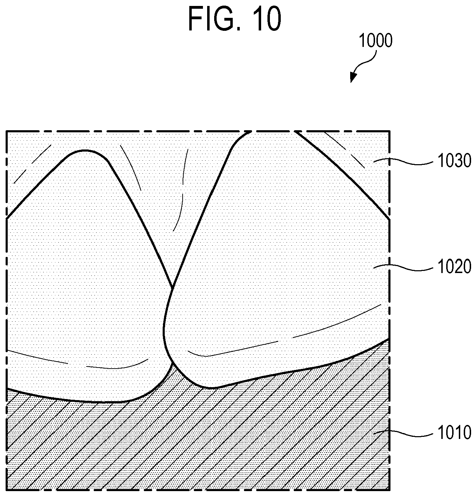

FIG. 10 illustrates an image in which a target object and a non-target object are separated and masked in an acquired two-dimensional image 1000 according to various embodiments of the present disclosure. As shown in FIG. 10, a portion 1010 other than a target object, that is, a soft tissue portion corresponding to the inside of the lips is separated from portions corresponding to a tooth 1020 and a gingiva 1030 and is masked. An embodiment of FIG. 10 shows a case where the mode for leaving the teeth and gingiva is selected, but the same method may be performed even when the mode for leaving only the teeth is selected. In an embodiment, the masked image may be displayed in the live view area 520 of the data acquisition program 500.

In step 950, when it is determined that the target object does not exist in the acquired two-dimensional image, the scan depth value is reduced.

In an embodiment, it is determined that the target object does not exist when the target object does not exist in the two-dimensional image acquired for a predetermined time or a predetermined number of scanning operations. A scan depth value may not be reduced when at least a portion of the target object is scanned in a two-dimensional image acquired for a predetermined time or for a predetermined number of scan operations.

In one embodiment, the reducing of the scan depth value includes reducing the scan depth value to an extent that scanning is substantially impossible. For example, the scan depth value may be changed to a minimum scan depth value (e.g., 0.01 mm). Alternatively, the scan depth value may be changed to 0 or less.

In an embodiment, in case that the scan depth value is reduced so that scanning is substantially impossible, the user may be informed of this through visual, auditory, and tactile notifications in various manners as described above.

When recognizing, through an alarm, that the scan depth is narrowed by reducing the scan depth value and practically no scan is performed, the user may move the scanner to a portion where the target object exists and restart the scan operation, or readjust the scan depth value to a value enabling scanning through a method for manipulating the three-dimensional scanner 200 or the scan depth control bar 570 of the data acquisition program 500, or the like. Through the configuration described above, when scanning of an unnecessary portion having no target object continues, the user is warned of the situation, thereby preventing an unnecessary scanning operation and data processing from continuing.

Various embodiments of the present disclosure may be implemented as software recorded in a machine-readable recording medium. The software may be software for implementing the above-mentioned various embodiments of the present disclosure. The software may be inferred from various embodiments of the present disclosure by programmers in a technical field to which the present disclosure belongs. For example, the software may be a machine-readable command (e.g., code or a code segment) or program. A machine may be a device capable of operating according to an instruction called from the recording medium, and may be, for example, a computer. In an embodiment, the machine may be the device 100 according to embodiments of the present disclosure. In an embodiment, a processor of the machine may execute a called command to cause elements of the machine to perform a function corresponding to the command. In an embodiment, the processor may be the at least one processor 101 according to embodiments of the present disclosure. The recording medium may refer to any type of recording medium which stores data capable of being read by the machine. The recording medium may include, for example, ROM, RAM, a CD-ROM, a magnetic tape, a floppy disk, an optical data storage device, and the like. In an embodiment, the recording medium may be the at least one memory 103. In an embodiment, the recording medium may be distributed to computer systems which are connected to each other through a network. The software may be distributed, stored, and executed in the computer systems. The recording medium may be a non-transitory recording medium. The non-transitory recording medium refers to a tangible medium that exists irrespective of whether data is stored semi-permanently or temporarily, and does not include a transitorily transmitted signal.

Although the technical idea of the present disclosure has been described by the examples described in some embodiments and illustrated in the accompanying drawings, it should be noted that various substitutions, modifications, and changes can be made without departing from the technical scope of the present disclosure which can be understood by those skilled in the art to which the present disclosure pertains. In addition, it should be noted that such substitutions, modifications, and changes are intended to fall within the scope of the appended claims.

## Claims

1. A method for processing a scan image of a three-dimensional scanner, which is performed by at least one processor of an electronic device comprising the at least one processor and at least one memory configured to store instructions to be executed by the at least one processor, the method comprising:
acquiring, through the three-dimensional scanner, scan data of a measurement object within a preconfigured scan depth of the three-dimensional scanner;
determining whether the scan data is abnormal based on a scan image acquired from the three-dimensional scanner; and
adjusting the scan depth of the three-dimensional scanner in a case of determining that there is abnormality in the scan data.

2. The method of claim 1, wherein the determining whether the scan data is abnormal based on a scan image acquired from the three-dimensional scanner comprises, in case that the measurement object does not exist within the scan depth or a density of the acquired scan image is equal to or less than a predetermined value, determining that the scan data is abnormal.

3. The method of claim 2, wherein the adjusting the scan depth comprises increasing a scan depth value of the three-dimensional scanner.

4. The method of claim 3, wherein the increasing the scan depth value comprises configuring the scan depth value to be a preconfigured scan depth value or increasing the scan depth value by a predetermined value.

5. The method of claim 1, wherein the determining whether the scan data is abnormal based on a scan image acquired from the three-dimensional scanner comprises, in case that noise data is included in the acquired scan image, determining that the scan data is abnormal.

6. The method of claim 5, wherein the adjusting the scan depth comprises reducing a scan depth value of the three-dimensional scanner.

7. The method of claim 3, wherein the reducing the scan depth value comprises configuring the scan depth value to be a preconfigured scan depth value or reducing the scan depth value by a predetermined value.

8. The method of claim 1, wherein the determining whether the scan data is abnormal based on a scan image acquired from the three-dimensional scanner comprises, in case that a target region is not included in the acquired scan image, determining that the scan data is abnormal .

9. The method of claim 8, wherein the adjusting the scan depth comprises configuring a scan depth value to be a minimum scan depth value.

10. The method of claim 1, wherein the adjusting the scan depth comprises providing a visual, auditory, or tactile notification to a user.

11. An electronic device comprising:
a communication circuit communicatively connected to a three-dimensional scanner;
a display; and
at least one processor,
wherein the at least one processor is configured to:
acquire, through the three-dimensional scanner, scan data of a measurement object within a preconfigured scan depth of the three-dimensional scanner;
determine whether the scan data is abnormal based on a scan image acquired from the three-dimensional scanner; and
adjust the scan depth of the three-dimensional scanner in a case of determining that there is abnormality in the scan data.

12. The electronic device of claim 11, wherein the determining whether the scan data is abnormal based on a scan image acquired from the three-dimensional scanner comprises, in case that the measurement object does not exist within the scan depth or a density of the acquired scan image is equal to or less than a predetermined value, determining that the scan data is abnormal.

13. The electronic device of claim 12, wherein the adjusting the scan depth comprises increasing a scan depth value of the three-dimensional scanner.

14. The electronic device of claim 13, wherein the increasing the scan depth value comprises configuring the scan depth value to be a preconfigured scan depth value or increasing the scan depth value by a predetermined value.

15. The electronic device of claim 11, wherein the determining whether the scan data is abnormal based on a scan image acquired from the three-dimensional scanner comprises, in case that noise data is included in the acquired scan image, determining that the scan data is abnormal.

16. The electronic device of claim 15, wherein the adjusting the scan depth comprises reducing a scan depth value of the three-dimensional scanner.

17. The electronic device of claim 13, wherein the reducing of the scan depth value comprises configuring the scan depth value to be a preconfigured scan depth value or reducing the scan depth value by a predetermined value.

18. The electronic device of claim 11, wherein the determining whether the scan data is abnormal based on a scan image acquired from the three-dimensional scanner comprises, in case that a target region is not included in the acquired scan image, determining that the scan data is abnormal.

19. The electronic device of claim 18, wherein the adjusting the scan depth comprises configuring a scan depth value to be a minimum scan depth value.

20. A non-transitory computer-readable recording medium for storing instructions configured to, when executed by at least one processor, cause the at least one processor to perform an operation,
wherein the instructions cause the at least one processor to:
acquire, through a three-dimensional scanner, scan data of a measurement object within a preconfigured scan depth of the three-dimensional scanner;
determine whether the scan data is abnormal based on a scan image acquired from the three-dimensional scanner; and
adjust the scan depth of the three-dimensional scanner in a case of determining that there is abnormality in the scan data.
